## Europäisches Patentamt
## European Patent Office
## Office européen des brevets

(19)

(11) Numéro de publication: **0 027 762**
**B1**

(12) **FASCICULE DE BREVET EUROPEEN**

(45) Date de publication du fascicule du brevet: **20.06.84**

(51) Int. Cl.³: **A 43 B 7/00**

(21) Numéro de dépôt: **80401475.1**

(22) Date de dépôt: **16.10.80**

(54) Bottillon anti-adductus articulé.

(30) Priorité: **18.10.79 FR 7925901**

(43) Date de publication de la demande:
**29.04.81 Bulletin 81/17**

(45) Mention de la délivrance du brevet:
**20.06.84 Bulletin 84/25**

(84) Etats contractants désignés:
**AT BE CH DE GB IT LI LU NL SE**

(56) Documents cités:
**FR - A - 431 782**
**US - A - 2 269 815**
**US - A - 2 967 360**
**US - A - 3 308 829**
**US - A - 3 413 977**
**US - A - 3 424 166**

(73) Titulaire: **ETABLISSEMENTS MAYZAUD Maurice**
**Rue Aimé-Cotton**
**Z.A.C. Brive Est FR-19100 Brive (FR)**

(72) Inventeur: **Mayzaud, Maurice**
**5 bis Rue Général Desbrulys**
**F-19100 Brive (FR)**

(74) Mandataire: **Chameroy, Claude et al,**
**c/o Cabinet Malemont 42, avenue du Président Wilson**
**F-75116 Paris (FR)**

Courier Press, Leamington Spa, England.

## Description

La présente invention concerne un bottillon anti-adductus articulé pour la correction active de l'adduction de l'avant-pied chez les nourrissons, bottillon dont la tige, qui est renforcée de manière à maintenir fermement en place l'arrière-pied du nourrisson, est fixée sur une demi-semelle postérieure et dont la partie antérieure, qui est destinée à envelopper étroitement l'avant pied déformé, est portée par une demi-semelle antérieure pouvant pivoter par rapport à la demi-semelle postérieure.

De nombreux nourrissons sont atteints d'une déformation de l'avant-pied appelée adduction, qui correspond à une déviation de l'avant-pied vers l'intérieur. Cette adduction peut s'observer dès la naissance du nourrisson, étant alors la conséquence d'une malformation intra-utérine, ou encore pendant les premiers mois de la vie, à la suite du décubitus ventral permanent chez les nourrissons, qui tend à pérenniser une déformation parfois négligée dès les premiers jours de vie. Cette adduction peut prendre des formes très sévères et rigides, a notamment dans le cas du pied métatarsus varus et du pied bot varus équin.

Diverses méthodes de kinésithérapie ont été proposées pour corriger ces formes sévères et rigides d'adduction. C'est ainsi qu'on connaît, pour la correction des pieds metatarsus varus, les attelles et plâtres de Kyte et, pour la correction des pieds bots varus équins, les manipulations et les attelles de Denis Brown. Cependant, même après ces traitements, il subsiste, dans le cas des formes sévères et rigides, une adduction résiduelle de l'avant-pied, qui nécessite un traitement correctif additionnel, et c'est dans le cadre de ce traitement additionnel, que les bottillons anti-adductus articulés, du type spécifié en préambule, trouvent leur utilisation la plus courante.

Le brevet américain n° 2967 360 décrit un tel bottillon. Dans ce bottillon connu la demi-semelle postérieure est rigidement fixée sur une semelle de base sur laquelle la demi-semelle antérieure est montée par l'intermédiaire d'un pivot. La semelle de base est en outre percée d'une fente en arc de cercle centrée sur le pivot. Cette fente est traversée par une vis qui prend appui contre la surface extérieure de la semelle de base et qui coopère avec un écrou tubulaire solidaire de la demi-semelle antérieure.

Pour effectuer une détorsion de l'avant-pied à l'aide de ce bottillon articulé, on place par pivotement la demi-semelle antérieure dans différentes positions angulaires successives rapprochées et on l'immobilise pendant plusieurs jours dans chacune de ces positions au moyen de l'ensemble vis-écrou. On réalise de la sorte une détorsion discontinue au cours de laquelle le flanc interne de la partie antérieure du bottillon ramène, par paliers successifs, l'avant-pied dans l'axe du pied.

L'inconvénient principal de ce bottillon anti-adductus connu est qu'il occasionne des douleurs aigües de l'avant pied, souvent insupportables pour le nourrisson, au début de chaque palier de détorsion. A cela, s'ajoute le fait qu'il peut être nécessaire de déchausser le nourrisson entre deux paliers successifs de détorsion.

Un autre inconvénient de ce bottillon connu, réside dans la présence d'une double semelle qui complique inévitablement sa fabrication et la rend plus lourd que les bottillons de marche classiques, ce qui peut être la cause d'une gêne importante pour le nourrisson. Par ailleurs, le fait que le pivot et l'ensemble vis-écrou soient situés sur la surface d'appui du pied, nécessite la mise en place, sur chacune des demi-semelles antérieure et postérieure, d'une semelle de confort avec interposition d'un coussinet de protection, ce qui complique encore plus la fabrication du bottillon.

On connaît par ailleurs, d'après le brevet américain n° 3424 166, une chaussure orthopédique pour adultes dont les deux demi-semelles sont reliées par un pivot formé de deux parties séparables pourvues de cannelures radiales, qui s'imbriquent l'une dans l'autre pour permettre là-encore une modification progressive dans le temps de la position angulaire relative des deux demi-semelles.

On retrouve, dans cette chaussure orthopédique, une grande partie des inconvénients mentionnés plus haut.

La présente invention se propose de remédier à ces inconvénients et, pour ce faire, elle a pour objet un bottillon anti-adductus articulé, du type spécifié en préambule, qui se caractérise en ce que ses deux demi-semelles sont reliées entre elles, du côté externe du bottillon, par au moins un ressort de rappel et sont articulées l'une sur l'autre autour d'un pivot suffisamment éloigné du ressort pour permettre à ce dernier de faire pivoter la demi-semelle antérieure vers l'extérieur, la partie antérieure du bottillon étant en outre rigidifiée sur le côté interne du bottillon.

On réalise ainsi, grâce à ce bottillon, une correction continue et efficace de l'adduction de l'avant-pied chez le nourrisson, la force de rappel du ressort tendant à reformer progressivement l'avant-pied par abduction pour le faire revenir définitivement dans l'axe du pied. Cette correction continue de l'adduction de l'avant-pied évite l'apparition de douleurs et s'effectue sans qu'il soit nécessaire de déchausser le nourrisson au cours du traitement.

Avantageusement, le pivot est situé le long du bord interne des deux demi-semelles et de préférence, au niveau de l'articulation de la première tête métatarsienne du pied. Cette disposition permet d'éviter au cours du traitement un allongement du bottillon, qui risquerait d'affecter l'efficacité de la détorsion.

Selon un mode préféré de réalisation de l'invention, les deux demi-semelles sont pourvues chacune, sur leur bord interne, d'un prolongement latéral, les deux prolongements latéraux se superposant et étant traversés tous deux par le pivot. De cette façon, le pivot se trouve en dehors de la surface d'appui du pied ce qui évite de prévoir des semelles de confort qui compliqueraient la fabrication du bottillon et par ailleurs favorise l'abduction sans allongement du bottillon.

Dans le même esprit, les deux demi-semelles comportent chacune une patte sur leur bord externe, les deux pattes étant espacées l'une de l'autre et reliées entre elles par le ressort.

Avantageusement, la tige est formée de deux parois latérales réunies à l'arrière de la tige et aptes à être reliées entre elles à l'avant de celle-ci par une attache auto-agrippante.

En complément, le bottillon selon l'invention comprend une sous-patte frontale destinée à fixée fermement entre les deux parois latérales de la tige par un système bride-boucle de fermeture afin d'exercer une pression sur le cou-de-pied. Le pied est de cette manière bloqué au fond du bottillon.

Par ailleurs, la partie antérieure du bottillon est constituée par un flanc latéral interne rigide et un flanc latéral externe fixés chacun sur un bord latéral de la demi-semelle antérieure et pouvant être réunis autour de l'avant-pied par une attache auto-agrippante.

Avantageusement, la tige est reliée au flanc latéral interne de manière à délimiter avec celui-ci une saignée arquée à fond plein, s'étendant sur sensiblement toute la hauteur du flanc latéral interne au-dessus de la zone d'articulation des demi-semelles et, est reliée au flanc latéral externe de manière à définir avec celui-ci une fente arquée s'évasant vers le bas sur la même hauteur au-dessus du ressort. Ainsi, tout en étant constitué de deux parties pivotantes l'une par rapport à l'autre, le bottillon selon l'invention se présente sous la forme d'une chaussure monobloc, la saignée permettant la flexion sans déformation du côté interne du bottillon au niveau de l'articulation.

Un mode de réalisation de l'invention est décrit ci-après à titre d'exemple, en référence au dessin annexé dans lequel:

La figure 1 est une vue en perspective d'un bottillon selon l'invention en position de repos,

La figure 2 est une vue de dessus du bottillon de la figure 1, et

La figure 3 est une vue de dessus représentant uniquement les deux demi-semelles assemblées, en traits pleins dans leur position de repos et, en traits mixtes dans la position de travail dans laquelle elles se trouvent lorsque l'adduction est corrigée.

En se référant simultanément au trois figures, il ressort que le bottillon selon l'invention est constitué par une tige 1 et une partie antérieure 2.

La tige 1 est constituée essentiellement par deux parois latérales externe 3 et interne 4 qui sont reliées sur le dos de la tige par une couture verticale non visible sur les dessins. Cette tige est fixée sur une demi-semelle postérieure 7. Cet ensemble constitue ainsi une coque rigide destinée à recevoir l'arrière-pied.

La partie antérieure 2 se compose de deux flancs externe 5 et interne 6 fixés sur les deux bords latéraux d'une demi-semelle antérieure 8. Les deux flancs 5 et 6 peuvent être rabattus l'un vers l'autre de manière à enserrer l'avant-pied à corriger. Le flanc interne est rigidifié pour efficacement participer à la détorsion de l'avant-pied.

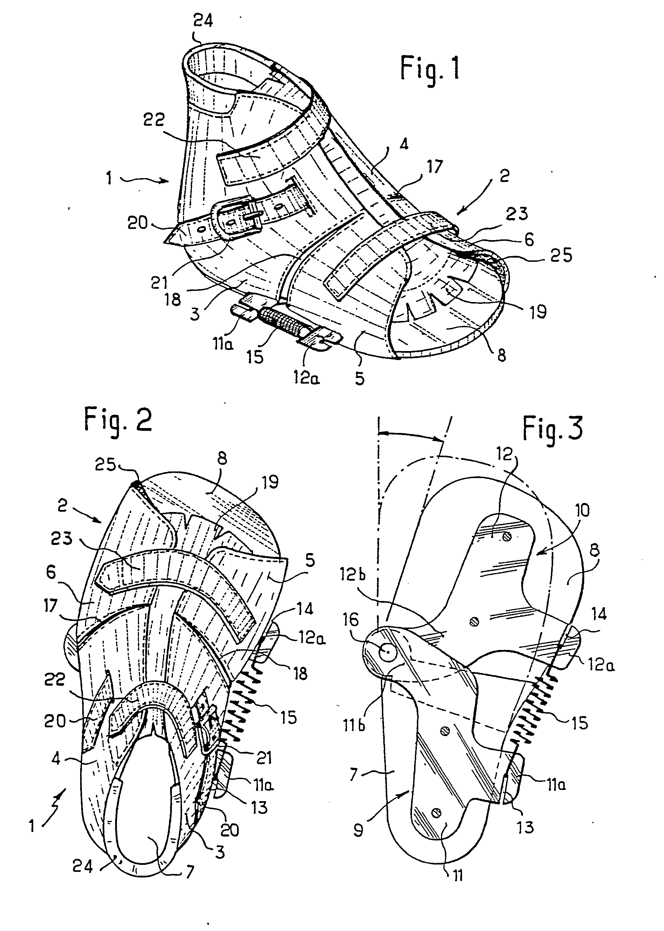

Les deux demi-semelles 7 et 8 sont renforcées chacune par une armature respectivement 9 et 10. Ces armatures qui sont constituées d'une matière dure sont chacune fixées normalement sous la demi-semelle correspondante, mais, pour plus de clarté, elles ont été représentées en traits pleins sur la figure 3. Chacune de ces armatures est formée par une branche centrale 11, 12 et deux branches latérales extérieures 11a, 12a et intérieures 11b, 12b.

Sur les deux branches latérales extérieures 11a et 12a sont pratiquées des encoches 13 et 14 qui reçoivent chacune une extrémité d'un ressort de rappel amovible 15. Celui-ci sollicite l'une vers l'autre les deux branches latérales extérieures 11a et 12a, et par suite les deux demi-semelles 7 et 8. Ce ressort 15 peut être de raideur différente suivant la gravité de l'adduction à corriger. Il est bien évident aussi que pour produire le même effet, plusieurs ressorts peuvent être montés en parallèle.

Les deux branches latérales intérieures 11b et 12b de chacune des armatures sont reliées l'une sur l'autre par un élément d'articulation 16 de manière à pouvoir pivoter l'une par rapport à l'autre en réponse à la sollicitation du ressort 15. L'élément d'articulation 16 est situé au niveau de l'articulation de la première tête métatarsienne du pied. Cette disposition suffit à faire pivoter l'une sur l'autre les deux demi-semelles. On peut néanmoins faire en sorte que les deux demi-semelles soient articulées elles-mêmes l'une sur l'autre en plus des armatures, comme on le voit sur la figure 3. A cet effet, chacune des demi-semelles comporte litéralement un prolongement que traverse l'élément d'articulation 16. Celui-ci peut être dans ce cas constitué par un premier rivet creux traversant les deux branches latérales intérieures 11b et 12b des armatures 9 et 10 de manière à ce que l'une de ces deux branches soit solidaire du rivet et l'autre fixée à rotation autour de celui-ci, et un second rivet s'emboîtant dans le premier et auquel les deux prolongements des deux demi-semelles sont fixés de la même manière que les armatures.

Au-dessus de l'élément d'articulation 16, la paroi latérale interne 4 de la tige 1 et le flanc interne 6 de la partie antérieure 2 sont reliés de manière à délimiter une saignée arquée 17 à fond plein. Ainsi la paroi latérale interne du bottillon peut suivre le pivotement les deux demi-semelles l'une sur l'autre sans déformation et sans exercer un effort sur la partie interne du pied située en arrière du premier métatarse.

Au-dessus du ressort 15, la paroi latérale externe 3 de la tige 1 et la flanc externe 5 de la partie antérieure 2 sont reliés de manière à délimiter une fente arquée 18 s'étendant sur une hauteur légèrement inférieure à celle du flanc 5 et s'évasant vers le bas.

Ainsi, lorsqu'elle est sollicitée vers l'intérieur par l'adduction de l'avant-pied, la partie antérieure 2 peut s'écarter de la tige 1 sans s'en séparer totalement. L'aspect unitaire du bottillon est ainsi conservé.

Le bottillon selon l'invention comprend en outre une sous-patte frontale de fermeture 19 qui peut être rembourrée intérieurement et qui est destinée à être fixée fermement entre les deux parois latérales de la tige 1 par une bride 20, solidaire d'une des parois latérales, et pouvant se refermer dans une boucle 21 solidaire de l'autre paroi latérale. En serrant cette bride 20, qui est engagée dans deux passants de la sous-patte 19 et dans deux autres passants des parois latérales 3 et 4, une pression est exercée sur le cou-de-pied et, de la sorte, la pied est fermement maintenu en place dans le fond du bottillon.

Afin de compléter l'immobilisation du pied dans le bottillon, deux bandes à auto-fermeture par contact de type "VELCRO" 22 et 23 sont prévues pour rapprocher respectivement les parois latérales 3 et 4 de la tige 1 et les flancs 5 et 6 de la partie antérieure 2 sur le cou-de-pied.

Lorsque le pied atteint d'adduction est enfilé et fermement immobilisé dans le bottillon, il est alors efficacement soumis à l'effort de sollicitation du ressort 15 vers l'extérieur. Progressivement, l'avant-pied revient dans la position axiale du pied, position représentée en traits mixtes sur la figure 3, à la suite du pivotement l'une par rapport à l'autre des deux demi-semelles qui peuvent à cet effet se chevaucher comme on le voit sur la figure 3.

Etant donné que le pied est soumis à des efforts importants, des éléments de protection sont aménagés au niveau des pressions exercées pour assurer le confort du pied et éviter toute allergie.

Ainsi, le sommet de la tige 1 est capitonné d'un bourrelet de mousse 24 ou de toute autre matière souple suffisamment compacte, qui protège le tendon d'Achille. Dans le même esprit, le flanc interne 6 de la partie antérieure 2 est renforcé par un coussinet de mousse 25 qui soulage l'avant-pied des efforts importants subis à cet endroit. Enfin, la demi-semelle postérieure 7 peut être recouverte d'un revêtement de mousse destiné à protéger le talon pressé par la bride de la sous-patte 19 contre le fond du bottillon.

On comprendra aisément que le bottillon selon l'invention permet une correction progressive et sans douleur pour le nourrisson de l'adduction de l'avant-pied dont il est atteint. Il peut s'intégrer avec succès dans les formes de traitement citées ci-dessus pour compléter la correction, soit simultanément, soit à la suite de celles-ci.

**Revendications**

1. Bottillon anti-adductus articulé pour la correction active de l'adduction de l'avant-pied chez les nourrissons, bottillon dont la tige (1), qui est renforcée de manière à maintenir fermement en place l'arrière-pied du nourrisson, est fixée sur une demi-semelle postérieure (7) et dont la partie antérieure (2), qui est destinée à envelopper étroitement l'avant-pied déformé, est portée par une demi-semelle antérieure (8) pouvant pivoter par rapport à la demi-semelle postérieure, caractérisé en ce que les demi-semelles (7, 8) sont reliées entre elles, du côté externe du bottillon, par au moins un ressort de rappel (15) et sont articulées sur l'autre autour d'un pivot (16) suffisamment éloigné du ressort (15) pour permettre à ce dernier de faire pivoter la demi-semelle antérieure vers l'extérieur, la partie antérieure (2) du bottillon étant en outre rigidifiée sur la côté interne du bottillon.

2. Bottillon selon la revendication 1, caractérisé en ce que le pivot (16) est situé le long du bord interne des deux demi-semelles (7, 8).

3. Bottillon selon la revendication 2, caractérisé en ce que le pivot (16) est situé au niveau de l'articulation de la première tête métatarsienne du pied.

4. Bottillon selon la revendication 2 ou 3, caractérisé en ce que les deux demi-semelles (7, 8) sont pourvues chacune, sur leur bord interne, d'un prolongement latéral, les deux prolongement latéraux (11b, 12b) se superposant et étant traversés tous deux par le pivot (16).

5. Bottillon selon l'une quelconque des revendications 1 à 4, caractérisé en ce que les deux demi-semelles (7, 8) comportent chacune une patte sur leur bord externe, les deux pattes (11, 12) étant espacées l'une de l'autre et reliées entre elles par le ressort (15).

6. Bottillon selon l'une quelconque des revendications 1 à 5, caractérisé en ce que la tige (1) est formée de deux parois latérales (3, 4) réunies à l'arrière de la tige et aptes à être reliées entre elles à l'avant de celle-ci par une attache auto-agrippante (22).

7. Bottillon selon la revendication 6, caractérisé en ce qu'il comprend une sous-patte frontale (19) destinée à être fixée fermement entre les deux parois latérales (3, 4) de la tige (1) par un système bride-boucle de fermeture (20, 21) afin d'exercer une pression sur le cou-de-pied.

8. Bottillon selon l'une quelconque des revendication 1 à 7, caractérisé en ce que sa partie antérieure (2) est constituée par un flanc latéral interne rigide (6) et un flanc latéral externe (5) fixés chacun sur un bord latéral de la demi-semelle antérieure (8) et pouvant être réunis autour de l'avant-pied par une attache auto-agrippante (23).

9. Bottillon selon la revendication 8, caractérisé en ce que la tige (1) est réliée au flanc latéral interne (6) de manière à délimiter avec celui-ci une saignée arquée à fond plein (17), s'étendant sur sensiblement toute la hauteur du flanc latéral interne (6) au-dessus de la zone d'articulation des demi-semelles (7, 8), et est réliée au flanc latéral externe (5) de manière à définir avec celui-ci une fente arquée (18) s'évasant vers le bas sur le même hauteur au-dessus de ressort (15).

**Patentansprüche**

1. Beweglich verbundener Anti-Adductus-Kurzschaftstiefel zur aktiven Korrektur der Adduktion des Mittelfußes bei Säuglingen, dessen Schaft (1), der so verstärkt ist, daß der Mittelfuß des Säuglings fest am Platz gehalten wird, auf einer hinteren Halbsohle (7) befestigt ist und dessen Vorderteil (2), der dazu bestimmt ist, den deformierten Mittelfuß eng zum umschließen, von einer vorderen Halbsohle (8) getragen ist, die relativ zur hinteren Halbsohle schwenkbar ist, dadurch gekennzeichnet, daß die Halbsohlen (7, 8) untereinander, aussenseitig am Kurzschaftstiefel, durch wenigstens eine Rückholfeder (15) verbunden sind und eine auf der andern um einen von der Feder (15) ausreichend entfernten Drehzapfen (16) angelenkt sind, um letztere die vordere Halbsohle nach außen schwenken zu lassen, wobei das Vorderteil (2) des Kurzschaftstiefels außerdem auf der Innenseite des Kurzschaftstiefels versteift ist.

2. Kurzschaftstiefel nach Anspruch 1, dadurch gekennzeichnet, daß der Drehzapfen (16) an der Innenkante der beiden Halbsohlen (7, 8) liegt.

3. Kurzschaftstiefel nach Anspruch 2, dadurch gekennzeichnet, daß der Drehpunkt (16) in Höhe der Anlenkung des Kopfes des ersten Mittelfußknochens liegt.

4. Kurzschaftstiefel nach Anspruch 2 oder 3, dadurch gekennzeichnet, daß die beiden Halbsohlen (7, 8) jede auf ihrer Innenkante mit einer seitlichen Verlängerung versehen sind, wobei die beiden seitlichen Verlängerungen (11b, 12b) sich überlagern und beide vom Drehzapfen (16) durchdrungen sind.

5. Kurzschaftstiefel nach irgend einem der Ansprüche 1 bis 4, dadurch gekennzeichnet, daß die beiden Halbsohlen (7, 8) jede eine Lasche auf ihrem Außenrand aufweisen, wobei die beiden Laschen (11, 12) Abstand voneinander halten und durch die Feder (15) miteinander verbunden sind.

6. Kurzschaftstiefel nach irgend einem der Ansprüche 1 bis 5, dadurch gekennzeichnet, daß der Schaft (1) von zwei Seitenwänden (3, 4) gebildet ist, die hinten am Schaft vereinigt sind und vorne durch eine selbstgreifende Befestigung (22) miteinander verbindbar sind.

7. Kurzschaftstiefel nach Anspruch 6, dadurch gekennzeichnet, daß er eine frontale Unterleglasche (19) umfaßt, dazu bestimmt, zwichen den beiden Seitenwänden (3, 4) des Schaftes (1) durch ein Riemen-Schnalle-Schließsystem (20, 21) fest fixiert zu werden, um auf den Rist des Fußes einen Druck auszuüben.

8. Kurzschaftstiefel nach irgend einem der Ansprüche 1 bis 7, dadurch gekennzeichnet, daß sein Vorderteil (2) aus einer steifen, innenseitigen Flanke (6) und einer außenseitigen Flanke (5) besteht, die jede auf einer Seitenkante der vorderen Halbsohle (8) befestigt sind und um den Mittelfuß durch eine selbstgreifende Befestigung (23) verbunden werden können.

9. Kurzschaftstiefel nach Anspruch 8, dadurch gekennzeichnet, daß der Schaft (1) so mit der innenseitigen Flanke (6) verbunden ist, daß mit ihr ein bogenförmiger Einschnitt (17) mit durchgehendem Grund begrenzt wird, der sich über praktisch die gesamte Höhe der innenseitigen Flanke (6) über der Anlenkzone der Halbsohlen (7, 8) erstreckt, und mit der außenseitigen Flanke (5) so verbunden ist, daß er mit dieser einen bogenförmigen Spalt (18) festlegt, der sich über die gleiche Höhe über der Feder (15) nach unten hin verbreitert.

**Claims**

1. A hinged anti-adductus bootee for the active correction of the adduction of the fore foot in infants, in which bootee the upper (1), which is reinforced so as to hold the rear foot of the infant firmly in position, is fixed to a rear half sole (7) and the front part (2), which is intended to closely surround the deformed fore foot, is supported by a front half sole (8) adapted to pivot with respect to the rear half sole, characterized in that the half soles (7, 8) are connected together, on the outer side of the bootee, by at least one return spring (15) and are hinged to one another about a pivot (16) spaced sufficiently apart from the spring (15) to allow this latter to cause the front half sole to pivot rearwardly, the front part (2) of the bootee being further rigidified on the internal side of the bootee.

2. The bootee according to claim 1, characterized in that the pivot (16) is situated along the internal edge of the two half soles (7, 8).

3. The bootee according to claim 2, characterized in that the pivot (16) is situated at the level of the joint of the first metatarsal head of the foot.

4. The bootee according to claim 2 or 3, characterized in that the two half soles (7, 8) are each provided, on their internal edge, with a lateral extension, the two lateral extension (11b, 12b) being superimposed and having the pivot (16) passing through both.

5. The bootee according to any one of claims 1 to 4, characterized in that the two half soles (7, 8) each comprise a lug on their external edge, the two lugs (11, 12) being spaced apart and connected together by the spring (15).

6. The bootee according to any one of claims 1 to 5, characterized in that the upper (1) is formed of two side walls (3, 4) joined together at the rear of the upper and adapted to be connected together at the front thereof by a self gripping fastening (22).

7. The bootee according to claim 6, characterized in that it comprises a front under lug (19) intended to be fixed firmly between the two side walls (3, 4) of the upper (1) by a strap-buckle closure system (20, 21) so as to exert a pressure on the instep.

8. The bootee according to any one of claims 1 to 7, characterized in that its front part (2) is formed by a rigid internal lateral side (6) and an external lateral side (5) each fixed to a lateral edge of the front half sole (8) and being connectable together about the fore foot by means of a self gripping fastening (23).

9. The bootee according to claim 8, characterized in that the upper (1) is connected to the internal lateral side (6), so as to define therewith an arcuate groove with solid bottom (17), extending over substantially the whole height of the internal lateral side (6) above the hinging zone of the two half soles (7, 8), and is connected to the external lateral side (5) so as to define therewith an arcuate slit (18) widening out towards the bottom over the same height above the spring (15).

Fig. 1

Fig. 2

Fig. 3